# EUROPEAN PATENT SPECIFICATION

(11) **EP 0 265 906 B1**
(45) Date of publication and mention of the grant of the patent: **19.04.1995**
(21) Application number: 87115766.5
(22) Date of filing: 27.10.1987
(51) Int. Cl.: A61F 13/00, A61L 15/32

(54) **Wound dressing**
Wundverband
Pansement

(30) Priority: 31.10.1986 JP 260002/86; 31.10.1986 JP 260003/86; 31.10.1986 JP 260142/86
(43) Date of publication of application: 04.05.1988
(73) Proprietor: NIPPON ZEON CO., LTD., Tokyo (JP)
(72) Inventor: Shioya, Nobuyuki, Yokohama-shi Kanagawa-ken (JP); Kuroyanagi, Yoshimitsu, Tokyo (JP); Koganeo, Yasumi, Kanagawa-ken (JP); Yoda, Ryuichiro, Yokohama-shi Kanagawa-ken (JP)
(74) Representative: TER MEER STEINMEISTER & PARTNER GbR

(56) References cited:
- EP-A- 0 100 148
- EP-A- 0 117 438
- DD-A- 231 987
- DE-A- 2 845 686
- DE-B- 2 248 813
- FR-A- 2 077 381
- FR-A- 2 377 205
- GB-A- 2 170 713
- US-A- 3 867 520

## Description

### BACKGROUND OF THE INVENTION

### Field of the Invention

The present invention relates to a wound dressing, and more particularly to a wound dressing for use in treating wounds, such as burn and trauma.

### Description of the Prior Art

So far has been developed a variety of dressings for use in treating skin defects extending over a wide area due to trauma or wound, and these are roughly grouped into three:
(1) Grafts, such as homogenous or heterogenous thick-split graft and human aminion;
(2) Reconstruction materials from bodily substances, such as collagen membrane (non-woven fabric) and fibrin membrane; and
(3) Synthetic high molecular materials, typically with a dual-layer structure of silicone film and nylon knitting.

Any of the three however has difficulties. (1) Grafts are advantageous in the aspects of being useful for controlling insensible perspiration and for preventing the transudation of body liquids while, from an immunological stand, they have disadvantageously a strong rejection, bringing a too short duration of dressing effect. As (2) reconstruction materials, in particular, a collagen material, owing to stable supply of it, is mostly used. Its antigenicity can be weakened by enzyme treatment but it is rapidly decomposed and absorbed in the body, and useless for a long time-lasting dressing. (3) The synthetic high molecular materials are absorbed in the body without being subjected to decomposition and thus are of low antigenicity. Their stable supply is possible and they are sterilizable. On the other hand, generally they present the problem of having an inadequate affinity to tissues.

Also materials of woven or sponge structure with a silicone film adhered to one surface so as to prevent bacteria from invading are favorable in the aspect that they absorb exudate from affected area, and fibrin is produced there (the so-called primary vital adhesion), and subsequently the spread of fibroblasts and capillaries occurs to form a firm adhesion of the dressing onto the wound (the so-called secondary vital adhesion). The difficulty however is encountered in the tendency observed under the silicone film towards aggregation of body fluid proteins, accompanied by a high danger of permitting bacteria already present on the wound surface to utilize them as nourishment for their propagation, resulting in hindering healing of the wound.

Countermeasure against such infection is at present taken of applying a cream or ointment containing local antimicrobial to the surface of the wound, and has been revealed in the application of different wound dressings to be not very useful for preventing invasion of foreign bacteria.

GB-A-2 170 713 discloses a wound dressing of a porous layer structure having affinity to tissues of organisms comprising a first portion being adapted to be placed on the wound surface and a second portion superimposed on the first portion and which may contain an antimicrobial agent.

### OBJECTS AND SUMMARY OF THE INVENTION

An object of the present invention is to remove the above-mentioned defects or shortcomings involved in the prior art and to provide a wound dressing useful for absorbing exudate from the wound to prevent stay of the exudate between the dressing material and the surface of the wound, permitting tissues to develop and capable of protecting against invasion of foreign bacteria, with the result of promoting therapeutic effects.

Another object of the present invention is to provide a wound dressing capable of preventing bacterial infection of the wound and transudation of body fluids, and thus enabling an earlier healing of the wound.

A further object of the present invention is to provide a wound dressing of a multilayer structure and containing a therapeutic agent so that the above-mentioned therapeutic effects can be taken securely and satisfactorily.

The first embodiment of the invention is a wound dressing of a porous layer having a good affinity to tissues of organisms comprising a first portion being adapted to be placed on the wound surface and a second portion superimposed on the first portion, which is characterized by said first portion having a reinforcing material embedded therein, having pores of 20 to 500 »m in diameter, said pores having diameters which decrease from the wound surface towards the second portion, and a thickness of 1 to 10 mm; and said second portion having pores of not more than 20 »m in diameter and a thickness of 0.5 to 5 »m.

According to a preferred embodiment, said porous layer is made from a polyamino acid.

Suitable polyamino acids for use in the invention are poly-α-amino acids having good affinity to tissues of organisms and soluble in solvents permitting freeze-drying, and typical examples are poly (L-leucine), poly (γ-benzyl-L-glutamate), copoly (L-lysine-L-leucine), copoly (L-lysine-L-glutamic acid), etc.

The wound dressing may preferably be composed of a sponge and the outer surface (crust) layers of such a polyamino acid.

The wound dressing is applied to the wound, with the sponge layer of 20 to 500 »m in pore diameters and 1 to 10 mm thickness on the surface of the wound. The pores in the sponge layer therefore are preferable to run from the wound surface to the interface of both layers and have decreasing diameters in the same direction. Pore diameters of up to 20 »m may result in poor development of tissue and insufficient absorption of exudate while pore diameters of 500»m or more may produce inadequate adhesiveness to the wound surface to allow exudate to stay, thus both ranges of pore diameters being unsuitable for treatment of the wound. Besides, thicknesses of the sponge layer of 1mm or less may result in poor development of tissue and insufficient absorption of exudate, and those of not less than 10mm may produce inconvenient handling, thus either range being unsuitable.

The outer surface layer of up to 20»m in pore diameter and 0.5 to 5»m thickness is to be positioned at the remote side to the wound surface when the wound dressing is applied to wound, and plays the role of preventing foreign bacteria from invading into the wound. If the pore diameter is 20»m or more, or the thickness is more than 0.5»m, the usefulness may be unsatisfactory. Thicknesses of 5»m or more may result in inadequate permeability to vapor and oxygen, accompanied by undesired effects.

The wound dressing is made, for example, by the following procedure: A poly-α-amino acid solution is poured into a specified vessel and converted into gel at room temperature. After the surface is dried with warm wind, the gel is cooled suddenly to a frozen state, and dried under vacuum. Thus a wound dressing consisting of a crust layer, or outer surface layer, and a sponge layer formed in the vessel is obtained.

The thus-obtained wound dressing is useful for absorbing exudate and for the production of fibrin, with the result of the so-called primary vital adhesion, and subsequently, owing to this, for the spread of fibroblasts and capillaries, resulting in the so-called secondary vital adhesion, thus contributing to an earlier healing of the wound. This implies that a wound dressing for treating a wound such as burn or trauma, in particular having sufficient adherence to the wound which will not allow exudate to stay between the dressing material and the surface of the wound, thus with the effects of promoting healing of the wound, can be provided.

As understood from the above-stated, the first embodiment of a wound dressing according to the invention is characterized by comprising a sponge layer of specified pore diameters and specified thickness, which is useful for effectively absorbing exudate to produce a firm primary vital adhesion to the surface of the wound, and which is useful, under these conditions, for a vigorous development of fibroblasts and capillaries, with a good effect of the secondary vital adhesion, and an outer surface layer of specified pore diameter and specified thickness, which controls insensible perspiration owing to its permeability to water vapor and prevents foreign bacteria from invading, thus contributing effectively to an earlier healing of the wound.

The second embodiment of the invention is a wound dressing of a porous layer structure having good affinity to tissues of organisms comprising a first portion being adapted to be placed on the wound surface and containing an antimicrobial agent in an amount of not more than 50 % by weight; and a second portion superimposed on the first portion and containing an antimicrobial agent in an amount of 10 to 80 % by weight; which is characterized by said first portion having a reinforcing material embedded therein, having pores with a diameter of 20 to 500 »m, said pores having diameters which decrease from the wound surface towards the second portion, and a thickness of 1 to 10 mm; and said second portion having pores with a diameter of not more than 20 »m and a thickness of 0.5 to 5 »m.

The second embodiment of wound dressing contains in the porous layer an antimicrobial agent capable of destroying bacteria present on the wound and of preventing infection due to invading of foreign bacteria. For achieving this, the antimicrobial agent is desired to be released constantly very little by very little. Generally speaking, medicine contained in a hydrophilic high molecular material is readily released when the material is swelled, and therefore, a long time-lasting release of medicine is unexpectable while the medicine is contained in a hydrophobic high molecular material, if the latter is of a film, is likewise readily released from the crystalline region of it, and through the influence of the resulting vacancies, the medicine in the inside of the film is readily released. Thus it is difficult to control the releasing rate. For solving the difficulty, according to the invention, the base material of the aforesaid porous layer is made especially from a hydrophobic poly-α-amino acid, with the effects of remarkably limiting circulation of liquid in the layer and as the result, enabling long duration of release of the medicine. It has been demonstrated that for example, sulfadiazine silver used as a medicine was released at an approximately constant rate from the wound dressing according to the invention in physiological saline solution over the period of about one month.

The second embodiment of wound dressing according to the invention has a unique construction of a porous layer to which attention is to be attracted, thus to attain the above-mentioned effects: it comprises the first portion to be on the wound surface and the second portion on the remote side to the wound surface, these being set in specified ranges of pore diameters, thickness and content of antimicrobial agent.

As to the first portion, which is very important for the absorption of exuadate (body fluids) from the wound and development of tissue, pore diameters of up to 20»m (or too small) may result in poor development of tissue and insufficient absorption of exudate while pore diameters of 500»m or more (or too greater) may produce inadequate adhesiveness to the wound surface to allow exudate to stay. Accordingly the range of pore diameters is 20 to 500»m, preferably 50 to 200»m, Besides, thicknesses of the first portion is 1 to 10mm, preferably 2 to 5mm, because thicknesses of 1mm or less is too thin to allow full development of tissues and thorough absorption of exudate, and those exceeding 10mm may result in inconvenience or disadvantages in handling and moldability.

On the other hand, regarding the second or the outermost portion, which is useful for preventing foreign bacteria from invading, the pore diameters exceeding 20»m allow bacteria to pass through them into the tissues. The pore diameter is desirable to be about 1»m as the lower limit from the aspect of requirement for allowing oxygen and water vapor to pass and controlling insensible perspiration. The thickness is not less than 0.5»m from the aspect of preventing bacteria from invading, and on the other hand, too great a thickness is unsuitable and therefore it should be not more than 5»m from the same reason as above-stated. In particularly, it is desirable to be 1 to 3 »m.

In the first portion of the aforesaid porous layer, the pores are desirable to run from the surface to be placed on the wound surface towards the second or outer surface portion and to have decreasing diameters in the same direction. The outer surface layer (the second portion) is desirable to have uniform film structure. This construction enables thorough absorption of exudate from the wound and well development of tissue and strictly preventing foreign bacteria from invading.

The second embodiment of wound dressing according to the invention contains an antimicrobial agent in the porous layer so that the antimicrobial agent may be little by little released. For realizing this, the first portion contains the antimicrobial agent in an amount of 0-50% by weight. The content should be 50 % or less by weight because otherwise inadequate pliability of the dressing material results. The amount is preferably 30 to 50% by weight. The second portion contains an antimicrobial agent in an amount of 10% to 80% by weight because contents of not more than 10% by weight are associated with an undesirable short duration of antimicrobial release while contents exceeding 80% by weight result in insufficient pliability. The contents of antimicrobial in the second portion is preferably 30 to 50% by weight.

Suitable poly-α-amino acids superior in affinity to tissues for use in the second embodiment of the present invention are poly(γ-benzyl-L-glutamate) (PBLG), poly(L-leucine), poly(N^{ε} -carbobenzoxy-L-lysine), and combinations of these may be useful. These, which are hydrophobic, readily polymerizable and soluble in benzene or dioxane allowing freeze drying under vacuum, are of good workability, and thus suitable to be molded.

Examples of local antimicrobial agents suitable for use in the second embodiment of wound dressing according to the invention are sulfadiazine silver, sulfadiazine zinc, sulfadiazine cerium, silver nitrate and gentamicin. The wound dressing according to the second invention is made from a mixture of a substance to be molded and an antimicrobial agent added thereto.

The second embodiment of wound dressing according to the invention may be made by a process comprising adding a predetermined amount of antimicrobial agent to a solution of a substance to be molded, as of poly-α-amino acid, pouring the resulting solution into a vessel or mold , and then subjecting to quenching and freeze drying under vacuum, so that a sheet-molded porous layer is produced.

In combination with the antimicrobial, other medicines such as vasoconstrictors for hemostasis and analgesic may be used to be contained in the porous layer.

The first and second embodiments of wound dressing according to the present invention may preferably be coated on at least one surface with a substance having a high affinity to tissues or accelerating wound healing, especially the surface to be on the wound surface, and this contributes to promotion of initial vital adhesion, prevention against the stay of exudate between the wound surface and the facing surface of dressing, and in turn earlier healing of the wound. In stead of the coating, a similar substance-containing layer overlayed on the porous layer may be formed. The coating or formation of the overlayed layer is followed by freeze-drying. Suitable substances for this purpose are enumerated serum proteins, such as fibrinogen, albumin, γ-globulin or fibronectin, collagen including atherocollagen, gelatin or mucopolysaccarides. The wound dressing may be impregnated with the substance having the high affinity.

Fibrinogen is a blood clotting factor which is converted by the action of thrombin into fibrin. Fibrin has a very good adhesiveness to fibroblasts and supports their growth. Coating of it to the surface of the dressing to be placed on the wound surface therefore produces effects of hemostasis and full vital adhesion, in turn contributing to healing. Like fibrin, collagen has a high adhesiveness to fibroblasts and can promote their growth, thus producing similar effects.

The first and second embodiments of wound dressing according to the present invention have a reinforcing material embedded in the porous layer. These wound dressings are obtained in the process comprising pouring a solution as of poly-α-amino acid to be molded, if desired, containing a predetermined amount of an antimicrobial agent added thereto, into a mold including a reinforcing material such as silicone gauze or nylon mesh therein, and then subjecting the mold to quenching and freeze drying under vacuum to mold a sheet of porous layer.

The reinforcing material embedded in the porous layer endows mechanical strength to the wound dressing of such a porous layer, and in addition plays an important part in peeling the porous layer off, for example, after treatment with the wound dressing for depths II and III burns over a certain period. Then the base substance may remain in the tissue but it can be decomposed and absorbed in the body. As understood from this, the first portion of the porous layer must have an appreciable thickness (1 to 10mm) as previously defined, otherwise the part closely adhered to tissue will also be removed from it.

Besides, the wound dressing is desired to be moderately flexible as function of displacement of the concerned body part or site. A poor flexibility can cause an accidental peeling off of the wound dressing. To endow such flexibility as stated, the reinforcing material is desirable to have more or less elasticity. For use in the present invention are suitable as reinforcing materials, natural fibers as of protein, cellulose and mineral, and others; synthetic fibers as of polyurethane, polyolefin, polyvinyl chloride, polyvinylidene chloride, polyamide, silicone, polyester, and others; metallic fibers as of stainless steel, copper, and others. These are useful particularly in the form of a mesh, such as a nylon mesh and silicone gauze.

Other objects, features and advantages of the invention will appear more fully from the following detailed description thereof taken in connection with the accompanying drawings.

### BRIEF DESCRIPTION OF THE DRAWINGS

The drawings show illustrative embodiments of the present invention:
Fig.1 is a fragmentary perspective view of an embodiment of the present invention; and
Fig.2 is a cross-sectional view of the embodiment of Fig.1 drawn to an enlarged scale.

### DESCRIPTION OF THE PREFERRED EMBODIMENTS

Embodiments of the present invention will be described in detail with reference to the accompanying drawings hereinafter:

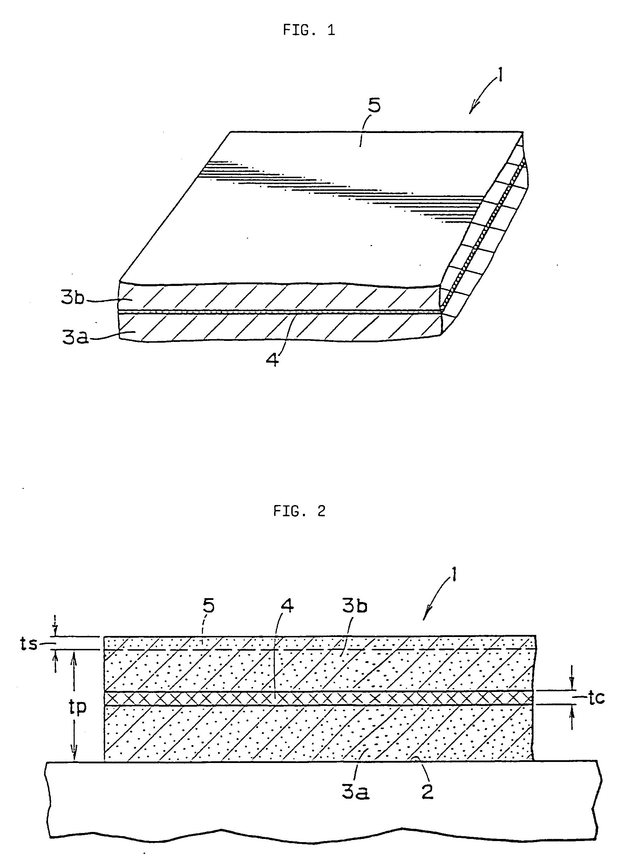

Fig.1 illustrates a fragmentary perspective view of a wound dressing 1 according to the invention and Fig.2 gives a cross-sectional view of the same. This dressing 1 consists of a lower porous portion 3 a to be closely adhered to the surface of a wound 2, an upper porous portion 3 b through the intermediation of reinforcing material 4 as of nylon mesh, and the outermost layer 5 of uniform structure having pores very small in diameter overlayed on the upper porous portion 3 b.

The porous portions 3 a, 3 b constituting the aforesaid first portion are made from poly-L-leucine and have a total thickness tₚ (herein the thickness of the embedded reinforcing material 4 is expressed as t_{c}) of 1 to 10mm, for example, about 2mm. Pores in these portions are of 20 to 500»m preferably 50 to 200»m, in diameters and formed to have decreasing diameter from the wound surface 2 towards the outer surface. The porous portions may contain a local antimicrobial agent, such as sulfadiazine silver at 50% by weight or less, as in the second embodiment of the present invention.

The embedded mesh-shaped material 4 for reinforcing the porous portion may be desirable to have t_{c} of 10 to 100»m and to be 1 to 2mm in mesh size. The polymer composing the portion gets in the mesh of the material 4.

The top portion 5 which corresponds to the above-stated second portion is made from poly-L-leucine and defined as represented by the dashed line and has a thickness tₛ of 0.5 to 5»m, preferably 1 to 3»m. Pores in this port ion 5 are formed to be very small or up to 20»m, such as several »m in diameter. This portion 5 may contain, as in the second embodiment of the invention, a local antimicrobial agent, such as sulfadiazine silver in an amount of 10 to 80% by weight, preferably 30 to 50% by weight.

The reinforcing material 4 may be embedded, for example, at about middle level in relation to the overall thickness of the dressing, for instance, 1mm deep when the overall thickness is 2mm, and located otherwise, for example, nearer to the outer surface or the surface to be on the wound surface, though the location is desirable at depths of about 0.5mm or more from either surface but not necessary to be so deep.

In the following Examples will be described.

### Reference Example 1.

N-carboxyl anhydride of L-leucine was dissolved in benzene and polymerized with triethylamine as an initiator. The poly(L-leucine) was dissolved in benzene to a concentration of 0.5 g/dl, and this poly(L-leucine) solution (30ml), after warmed to 70°C (because it is a gel at room temperature), was poured into an aluminum vessel (10cm x 15cm). After it gel led at room temperature, only the surface of the gel was dried with warm wind having about 50°C. Then benzene was evaporated to make the concentration of polymer in the gel higher, and in turn cooling at -30°C to frozen state and freeze drying under vacuum were conducted to obtain a wound dressing. This product was of multilayer structure consisting of a uniform (substantially unmeasurable pore diameter) crust layer of about 14»m thick and a sponge layer of about 2.4mm thick and having decreasing pore diameters from 300 to 400»m at the surface to be adhered to the wound surface to 50 to 100»m at the surface bounding the crust layer.

This wound dressing was evaluated in 6-8 weeks age rats: Each rat was subjected to surgery in the dorsal region on one side to create there full thickness skin defect (3cm x 2.5cm). The wound dressing was applied to it and sutured. Then gentamicin ointment was applied to the circumference, Telfa pad was sutured and elastic bandage was applied.

At 4 weeks after the application of such dressing, the dressing was removed together with the surrounding tissue, stained with hematoxylin-eosin, and examined histologically with the result of good vital adhesion, and the findings were obtained showing development of tissues into the sponge, poor appearance of foreign body giant cell, and, in the lower zone of the sponge layer, new tissues with abundant in capillaries. The dressing was peeled off with minor bleeding, and the tissue surface observed had good appearance, and somewhat dry slate suggesting it for insensible perspiration to be kept controlled.

### Comparison Example 1.

In the same way as in Reference Example 1, a wound dressing was obtained with a solution of poly(L-leucine) in benzene, excluding drying of the surface with warm wind of 50°C. The dressing had no crust but only a single layer of sponge. The thickness is 2.5mm and the pore diameter at the surface to be adhered to wound surface was 300 to 400»m.

### Comparison Example 2.

The same solution as used in Reference Example 1, of poly(L-leucine) in benzene, was poured into an aluminum vessel, changed into gel at room temperature, and then the whole was dried with warm wind having about 50°C. In turn, quenching to frozen state of -30°C, and freeze-drying under vacuum followed. The thus-obtained wound dressing comprised a single layer of the same phase as the above-mentioned crust layer, and the thickness was 1mm.

The wound dressings obtained in Comparison Examples 1 and 2 were evaluated in rats in the same way as in Reference Example 1. The product of Comparison Example 1 showed the surface remaining inflamed and having poor development of new tissues, this being probably due to invasion of foreign bacteria.

Examination of the wound dressing of Comparison Example 2 revealed stay of exudate and neither vital adhesion nor development of new tissue. This is considered to be due to absence of sponge layer allowing development of tissues thereinto, resulting in poor vital adhesion, and in turn insufficient absorption of body liquid, accordingly stay of it, on the wound surface.

### Reference Example 2.

A mixture of poly(L-leucine) With sulfadiazine silver was dissolved in benzene to make a concentration of 0.25g/dl, and poured into an aluminum vessel. The polymer solution was, after only the surface was dried with warm wind, quenched at -30°C, and subjected to freeze-drying under vacuum to obtain a sheet-molded wound dressing.

This wound dressing may be gas-sterilized and be kept in darkness. Or it was coated with a substance having good affinity to tissue or promoting healing of wound in the procedure: it was immersed in ethanol , and washed with sterilized distilled water. On the layer 3 of the wetted wound dressing, an aqueous solution of human fibrinogen (concentration 1 g/dl) was coated. Subsequently quenching to -20°C, freeze-drying, and ultraviolet ray radiation in a sterile room for several hours were in turn carried out, and the product may be kept at 5°C in darkness.

This wound dressing was evaluated in 6-8 weeks age rats: Each rat was subjected to surgery in the dorsal region on one side to create there a full thickness skin defect (3cm x 2.5cm). Then the wound dressing was applied to it and sutured and gentamicin ointment was applied to the circumference, Telfa pad was sutured, and elastic bandage was applied there. The application of the wound dressing was done merely by light press over about 1 minute with result of moderate adhesion, so that the suture of the wound dressing was carried out without allowing it to move. This may be probably owe to the adhesive action of fibrin converted from fibrinogen in the lower portion of the layer. It demonstrated well hemostatic effect on such minor bleeding as oozing on the wound surface. The adhesive and hemostatic effects at the initial may more likely contribute to prevention against ecchymoma underneath the dressing. At 2 and 4 weeks were obtained histological findings showing good vital adhesion caused and development of new tissue abundant in capillaries in the lower portion of the layer.

### Example 1

A mixture of poly(L-leucine) with sulfadiazine silver was dissolved in benzene to make a concentration of 0.25g/dl), and poured into an aluminum vessel having a nylon mesh previously set at a level of 1mm from the bottom. The polymer solution was, after only the surface was dried with warm wind, quenched at -30°C, and subjected to freeze-drying under vacuum to obtain a sheet-molded wound dressing.

This wound dressing was gas-sterilized, and coated with a substance having good affinity to tissue in the same way as in Reference Example 2. Evaluation of this wound dressing was made in 6-8 weeks age rats with the similar good results: Each rat was subjected to surgery in the dorsal region to create a full thickness skin defect. Then the wound dressing was applied to it merely by light press over about 1 minute, with result of moderate adhesion, accordingly without allowing it to move. It demonstrated well hemostatic effect on such minor bleeding as oozing on the wound surface. At 2 and 4 weeks were obtained histological findings showing good vital adhesion caused and development of new tissue abundant in capillaries in the lower portion of the layer.

It will be evident that various modifications can be made to the described embodiments without departing from the scope of the present invention as defined by the claims.

## Claims

1. A wound dressing of a porous layer structure having a good affinity to tissues of organisms comprising a first portion (3) being adapted to be placed on the wound surface (2) and a second portion (5) superimposed on the first portion (3), **characterized by** said first portion (3) having a reinforcing material embedded therein, having pores of 20 to 500 »m in diameter, said pores having diameters which decrease from the wound surface (2) towards the second portion (5), and a thickness of 1 to 10 mm; and said second portion (5) having pores of not more than 20 »m in diameter and a thickness of 0.5 to 5 »m.

2. The wound dressing claimed in claim 1, wherein said porous layer is made from a polyamino acid.

3. The wound dressing as claimed in claim 1, wherein said first portion (3) of said porous layer is made of a sponge.

4. The wound dressing as claimed in claim 1, wherein said reinforcing material is flexible.

5. The wound dressing as claimed in claim 1, wherein the surface of said porous layer to be on the wound surface (2) is coated or impregnated with a substance having an affinity to tissues of organisms.

6. A wound dressing of a porous layer structure having good affinity to tissues of organisms comprising a first portion being adapted to be placed on the wound surface and containing an antimicrobial agent in an amount of not more than 50 % by weight; and a second portion (5) superimposed on the first portion (3) and containing an antimicrobial agent in an amount of 10 to 80% by weight; **characterized by** said first portion (3) having a reinforcing material embedded therein, having pores with a diameter of 20 to 500 »m, said pores having diameters which decrease from the wound surface (2) towards the second portion (5), and a thickness of 1 to 10 mm; and said second portion (5) having pores with a diameter of not more than 20 »m and a thickness of 0.5 to 5 »m.

7. The wound dressing as claimed in claim 6, wherein said porous layer is made from a polyamino acid.

8. The wound dressing as claimed in claim 6, wherein said first portion (3) of said porous layer is made of a sponge.

9. The wound dressing as claimed in claim 6, wherein said reinforcing material is flexible.

10. The wound dressing as claimed in claim 6, wherein the surface of said porous layer to be on the wound surface (2) is coated or impregnated with a substance having an affinity to tissues of organisms.

11. The wound dressing as claimed in claim 6, wherein said antimicrobial agent is contained therein in combination with at least one therapeutic agent.

## Patentansprüche

1. Wundverband aus einer porösen Schichtstruktur mit einer guten Affinität gegenüber Geweben von Organismen, umfassend einen ersten Bereich (3), welcher zur Auflage auf die Wundoberfläche (2) angepaßt ist, und einen zweiten Bereich (5), welcher über dem ersten Bereich (3) angeordnet ist, **dadurch gekennzeichnet**, daß der erste Bereich (3) darin eingebettet ein Verstärkungsmaterial aufweist, Poren mit einem Durchmesser von 20 bis 500 »m besitzt, wobei die Poren Durchmesser aufweisen, welche von der Wundoberfläche (2) zum zweiten Bereich (5) hin abnehmen, und eine Dicke von 1 bis 10 mm besitzt; und der zweite Bereich (5) Poren mit einem Durchmesser von nicht mehr als 20 »m aufweist und eine Dicke von 0,5 bis 5 »m besitzt.

2. Wundverband nach Anspruch 1, wobei die poröse Schicht aus einer Polyaminosäure hergestellt ist.

3. Wundverband nach Anspruch 1, wobei der erste Bereich (3) der porösen Schicht aus einem Schwamm hergestellt ist.

4. Wundverband nach Anspruch 1, wobei das Verstärkungsmaterial flexibel ist.

5. Wundverband nach Anspruch 1, wobei die Oberfläche der porösen Schicht, welche auf die Wundoberfläche (2) kommt, mit einer Substanz, welche eine Affinität gegenüber Geweben von Organismen besitzt, beschichtet oder imprägniert ist.

6. Wundverband aus einer porösen Schichtstruktur mit guter Affinität gegenüber Geweben von Organismen, umfassend einen ersten Bereich, welcher zur Auflage auf die Wundoberfläche angepaßt ist und ein antimikrobielles Mittel in einer Menge von nicht mehr als 50 Gew.-% enthält; und einen zweiten Bereich (5), welcher über dem ersten Bereich (3) angeordnet ist und ein antimikrobielles Mittel in einer Menge von 10 bis 80 Gew.-% enthält, **dadurch gekennzeichnet**, daß der erste Bereich (3) darin eingebettet ein Verstärkungsmaterial aufweist, Poren mit einem Durchmesser von 20 bis 500 »m besitzt, wobei die Poren Durchmesser aufweisen, welche von der Wundoberfläche (2) zum zweiten Bereich (5) hin abnehmen, und eine Dicke von 1 bis 10 mm besitzt; und daß der zweite Bereich (5) Poren mit einem Durchmesser von micht mehr als 20 »m aufweist und eine Dicke von 0,5 bis 5 »m besitzt.

7. Wundverband nach Anspruch 6, wobei die poröse Schicht aus einer Polyaminosäure hergestellt ist.

8. Wundverband nach Anspruch 6, wobei der erste Bereich (3) der porösen Schicht aus einem Schwamm hergestellt ist.

9. Wundverband nach Anspruch 6, wobei das Verstärkungsmaterial flexibel ist.

10. Wundverband nach Anspruch 6, wobei die Oberfläche der porösen Schicht, welche auf die Wundoberfläche (2) kommt, mit einer Substanz, welche eine Affinität gegenüber Geweben von Organismen besitzt, beschichtet oder imprägniert ist.

11. Wundverband nach Anspruch 6, wobei das antimikrobielle Mittel darin in Kombination mit mindestens einem therapeutischen Mittel enthalten ist.

## Revendications

1. Pansement ayant une structure à couche poreuse, ayant une bonne affinité pour les tissus d'organismes, comprenant une première partie (3) adaptée pour être placée sur la surface lésée (2) et une deuxième partie (5) superposée sur la première partie (3), caractérisé en ce que cette première partie (3) comporte un matériau de renforcement incorporé dans celle-ci, ayant des pores d'un diamètre de 20 à 500 »m, ces pores ayant un diamètre diminuant à partir de la surface lésée (2) vers la deuxième partie (5), et ayant une épaisseur de 1 à 10 mm ; et la deuxième partie (5) ayant des pores d'un diamètre non supérieur à 20 »m et une épaisseur de 0,5 à 5 »m.

2. Pansement selon la revendication 1, dans lequel la couche poreuse est réalisée en un poly(aminoacide).

3. Pansement selon la revendication 1, dans lequel la première partie (3) de cette couche poreuse est réalisée en une éponge.

4. Pansement selon la revendication 1, dans lequel le matériau de renforcement est souple.

5. Pansement selon la revendication 1, dans lequel la surface de la couche poreuse destinée à être sur la surface lésée (2), est revêtue ou imprégnée avec une substance ayant une affinité pour les tissus d'organismes.

6. Pansement ayant une structure à couche poreuse, ayant une bonne affinité pour les tissus d'organismes, comprenant une première partie adaptée pour être disposée sur la surface lésée, et contenant un agent antimicrobien selon une quantité non supérieure à 50 % en poids ; et une deuxième partie (5) superposée sur la première partie (3), et contenant un agent antimicrobien selon une quantité de 10 à 80 % en poids, caractérisé en ce que la première partie (3) comprend un matériau de renforcement incorporé dans celle-ci, et a des pores d'un diamètre de 20 à 500 »m, ces pores ayant un diamètre décroissant à partir de la surface lésée (2) vers la deuxième partie (5), et une épaisseur de 1 à 10 mm ; et la deuxième partie (5) comporte des pores d'un diamètre non supérieur à 20 »m, et a une épaisseur de 0,5 à 5 »m.

7. Pansement selon la revendication 6, dans lequel la couche poreuse est réalisée en un poly(aminoacide).

8. Pansement selon la revendication 6, dans lequel la première partie (3) de la couche poreuse, est réalisée en une éponge.

9. Pansement selon la revendication 6, dans lequel le matériau de renforcement est souple.

10. Pansement selon la revendication 6, dans lequel la surface de la couche poreuse destinée à être sur la surface lésée (2), est revêtue ou imprégnée avec une substance ayant une affinité pour les tissus d'organismes.

11. Pansement selon la revendication 6, dans lequel l'agent antimicrobien est contenu dans celui-ci, en association avec au moins un agent thérapeutique.
